# EUROPEAN PATENT SPECIFICATION

(11) **EP 1 077 683 B1**
(45) Date of publication and mention of the grant of the patent: **11.12.2002**
(21) Application number: 99921726.8
(22) Date of filing: 07.05.1999
(51) Int. Cl.: A61K 9/22, A61K 47/08, A61K 47/38, A61M 31/00, C08F 226/10

(54) **ANTIDEPRESSANT THERAPY**
THERAPIE VON DEPRESSIONEN
TRAITEMENT ANTIDEPRESSEUR

(30) Priority: 14.05.1998 US 85432 P
(43) Date of publication of application: 28.02.2001
(73) Proprietor: ALZA CORPORATION, Mountain View, CA 94043 (US)
(72) Inventor: AYER, Atul, D., Palo Alto, CA 94303 (US); SEROFF, Sylvia, Sunnyvale, California 94086 (US); WONG, Patrick, S.-L., Burlingame, CA 94010 (US)
(74) Representative: Suèr, Steven Johannes
(86) International application number: US9909917
(87) International publication number: WO99058115

(56) References cited:
- EP-A1- 0 640 341
- EP-B1- 0 132 384
- US-A- 4 612 008
- US-A- 5 324 280
- US-A- 5 591 454

## Description

### FIELD OF THE INVENTION

This invention pertains to an improvement in a dosage form comprising an antidepressant drug. The invention concerns also a pharmaceutical composition comprising an antidepressant drug and a pharmaceutical carrier for the antidepressant drug. The invention relates further to a method for the treatment of depressive affective disorders in a patient.

### BACKGROUND OF THE INVENTION

Antidepressant drugs are a recognized therapeutically accepted class of drugs used for the treatment of depression, particularly endogenous depression and sometimes reactive depression. One class of antidepressant drug used for these therapies is the tricyclic antidepressant drugs. The tricyclic antidepressant's efficacy in alleviating depression is clinically established in the management of health. Also, there is clinical support for the use of the tricyclic antidepressants in psychiatric disorders, such as in the management of depression accompanied by anxiety or agitation and panic attacks.

The antidepressant tricyclic drugs comprise an annealated three-ring nucleus, a tricyclic nucleus, which can comprise a member selected from the group consisting of dibenzazepinyl, dibenzocycloheptadienyl, dibenzoxepinyl, and phenothiazinyl. A further common structural moiety present in these antidepressant tricyclic drugs is the presence of a side chain bonded to an atom of the tricyclic ring, such as a member selected from the group consisting of carbon and nitrogen in the central ring of the tricyclic nucleus. The antidepressant tricyclic drugs are known in The Pharmacological Basis of Therapeutics, by Gilman and Rail, 8th Ed., pages 405 to 414, 1990, published by Pergamon Press, Inc.

In administering an antidepressant to a patient in need of antidepressant therapy, it is necessary to know that an effective dose of the drug was administered to produce the intended therapy. Additionally, in administering a tricyclic antidepressant to a patient in need of selected antidepressant therapy, it is necessary to know the administration of the antidepressant is at a controlled rate in a predetermined concentration range per unit time to ensure an effective dose was administered for good therapy and to essentially prevent overdosage and to minimize undesirable effects.

The prior art knew of the need for a dosage form endowed with controlled release properties for administering an antidepressant drug. For instance, in the prior art patent, United States No. 4,783,337 issued to patentees Wong, Barclay, Deters and Theeuwes, there is disclosed a dosage form that can contain amitriptyline and imipramine, useful for antidepressant therapy. While the prior art taught the antidepressants for their intended purpose, serious shortcomings have been found to exist in the prior art associated with tricyclic antidepressant drugs. For example, dosage forms, comprising a tricyclic structured antidepressant drug, often are sticky and they tend to clog the exit orifice of the dosage form; also, during manufacture the tricyclic antidepressant drugs tend to stick to the manufacturing machinery and this unwanted feature makes it difficult to provide operable and reliable dosage forms. Collectively, these shortcomings seriously hinder the manufacture of and the dispensing of a tricyclic antidepressant from a controlled release dosage forms. Additionally, the prior art did not disclose a more preferred dosage form comprising an antidepressant composition that can be administered for a chosen therapy.

US. patent number 4,612,008 discloses an osmotic system comprising a wall formed in at least a part of a semipermeable material that surrounds a compartment, the compartment containing a first osmotic composition comprising a beneficial agent, and a second expandable osmotic composition for dispensing the beneficial agent from the device.

US patent number 5,324,280 discloses an osmotic system for delivering a beneficial agent, which comprises an outside semipermeable wall, a middle osmotically active layer, a capsule comprising a beneficial agent, and a passageway for dispensing the beneficial agent from the osmotic system.

US patent number 5,591,454 discloses a method for administering the antidiabetic drug glipizide from a dosage form which comprises an osmagent and a hydrogel.

In the light of the above presentation, it will be self-evident to those versed in the dispensing art, that a pressing need exists for a dosage form that overcome the shortcomings of the prior art and possess a dosage form controlled rate of release that can deliver the valuable antidepressant drugs for their therapy. A dosage form made with rate controlled antidepressant delivery properties, is needed because it provides antidepressant drug at a controlled rate of release of the tricyclic drug versus time shows a substantially straight line that indicates the rate of release is independent of time. The pressing need exists for an essentially trouble-free dosage form characterized by a controlled rate of antidepressant release, which release is generated by activity by the dosage form, while simultaneously maintaining the improved physical and improved chemical integrity of the dosage form during the antidepressant drug delivery period.

### OBJECTS OF THE INVENTION

Accordingly, in view of the above presentation, it is an immediate object of this invention to provide a dosage form for delivering, at an essentially controlled rate of release, an antidepressant drug to a patient in need of antidepressant drug therapy.

Another object of the present invention is to provide a sustained-release dosage form that substantially overcomes the deficiencies and shortcomings associated with the prior art.

Another object of the present invention is to provide a sustained-release dosage form for administering an antidepressant in a known dose per unit time over a prolonged period of time up to thirty hours to provide a plasma concentration of the antidepressant.

Another object of the present invention is to provide a novel solid dosage form characterized by delivering a tricyciic antidepressant drug at a substantially sustained-release and controlled therapeutic rate for psychopharmacological antidepressant activity.

Another object of the invention is to provide a unique dosage form manufactured as an osmotic dosage form characterized by the dosage form delivering a tricyclic antidepressant drug to a biological receptor site to produce the desired pharmaceutical effects.

Another object of the invention is to provide a therapeutic composition of matter comprising an antidepressant tricyclic drug and pharmaceutically acceptable composition-forming ingredients in a ratio that enhances the delivery of the drug without restricting or reducing its delivery from the composition of matter.

Another object of the invention is to provide a dosage form comprising an antidepressant tricyclic drug and pharmaceutically-acceptable compositional components in proportions that lessens or prevents clogging the delivery exit of the dosage form thereby enabling the dosage form to deliver the therapeutic dose.

Another object of the invention is to provide a solid dosage form designed as a pharmaceutical tablet comprising an antidepressant tricyclic composition, which composition produces hydrodynamic/osmotic pressure for delivering the antidepressant from the dosage form over a prolonged time up to 24 hours.

Another object of the present invention is to provide a dosage form adapted for oral administration of a tricyclic antidepressant drug, which dosage form is characterized by comprising a composition containing at least one tricyclic antidepressant drug that is delivered in a known concentration per unit time to a patient.

Another object of the present invention is to provide a dosage form adapted for administering at least one tricyclic antidepressant drug, which dosage form is characterized by comprising a first tricyclic antidepressant composition and a second space consuming composition, which first and second composition act together for the sustained-release and rate controlled administration of a given dose of a tricyclic antidepressant drug to a patient over thirty hours.

Another object of the present invention is to provide a composition of matter comprising a microencapsulated tricyciic antidepressant drug, which composition is useful for manufacturing a dosage form.

Another object of the present invention is to provide a complete pharmaceutical regimen comprising an administrable composition, which composition comprises a microencapsulated tricyclic antidepressant drug and which composition can be dispensed from an osmotic delivery device, the use of which delivery device requires intervention only for initiation and possibly for the termination of the regimen.

Another object of the invention is to provide a method of using a dosage form possessing osmotic properties for use in a method of administering a polymeric-encapsulated tricyclic-antidepressant drug for the management of mental depression.

Another object of the invention is to provide a method for treating depression, accompanied by anxiety, comprising administering an antidepressive effective dose of a polymer-encased antidepressant tricyclic drug from a substantially zero order delivery dosage form, to a warm-blooded animal, including humans.

Another object of the invention is to provide a method for the relief of symptoms of depression, including endogenous depression, by administering an antidepressive effective dose of an antidepressive tricyclic drug from an osmotically activated dosage form, to a patient in need of therapy.

Another object of the present invention is to provide the use of a dosage form for administering a tricyclic antidepressant from a dosage form at a controlled rate over time, wherein the tricyclic antidepressant is selected from the group consisting of a base, an acceptable salt, a racemate, and isomers.

Another object of the present invention is to provide a composition of matter comprising an antidepressant tricyciic drug surrounded by a polymer in inventive proportions for unexpected therapy.

Another object of the present invention is to provide a tricyclic antidepressant drug encapsulated by a composition comprising a polymer for substantially lessening the incidence of stickiness associated with a tricyclic antidepressant drug.

Another object of the present invention is to substantially decrease and/or prevent blocking of an exit orifice of a dosage form by administering an encapsulated tricyclic antidepressant drug from the dosage form

Another object of the present invention is to increase the manufacturing effectiveness of a process for manufacturing a dosage form comprising a tricyclic-antidepressant drug microencapsulated for lessening and/or preventing the antidepressant drug from sticking to manufacturing machinery.

Other objects, features and advantages of the invention will be more apparent to those versed in the dispensing acts, from the following detailed specification, taken in conjunction with the drawings and the accompanying claims.

### BRIEF DESCRIPTION OF THE DRAWINGS

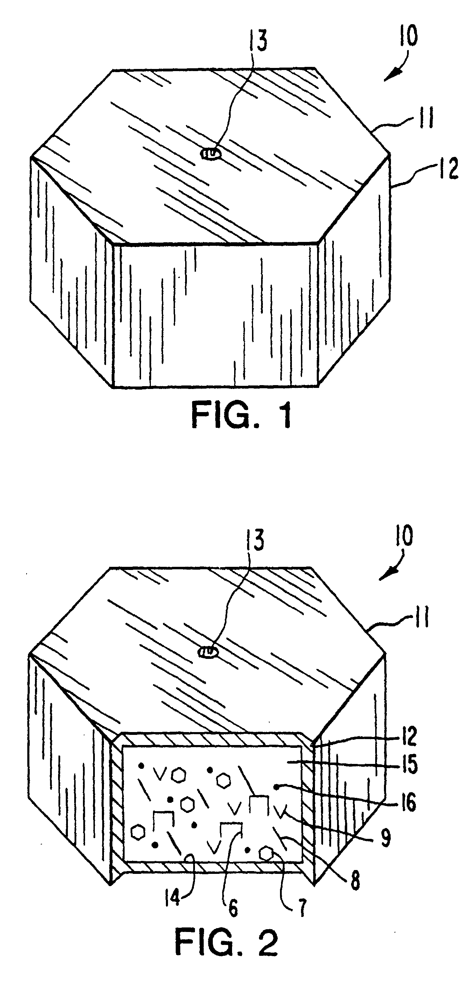

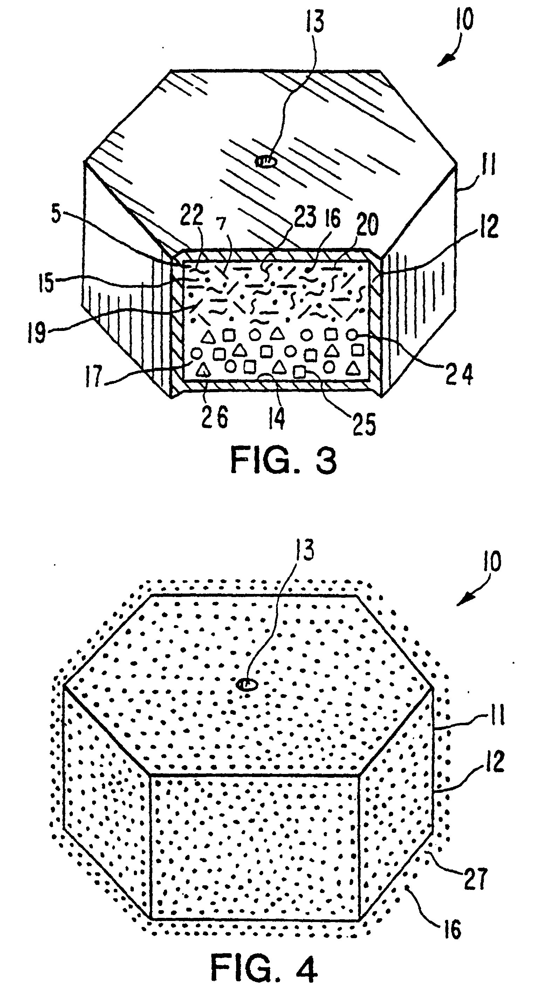

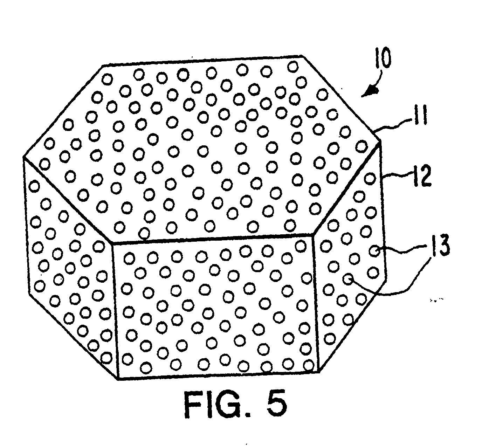

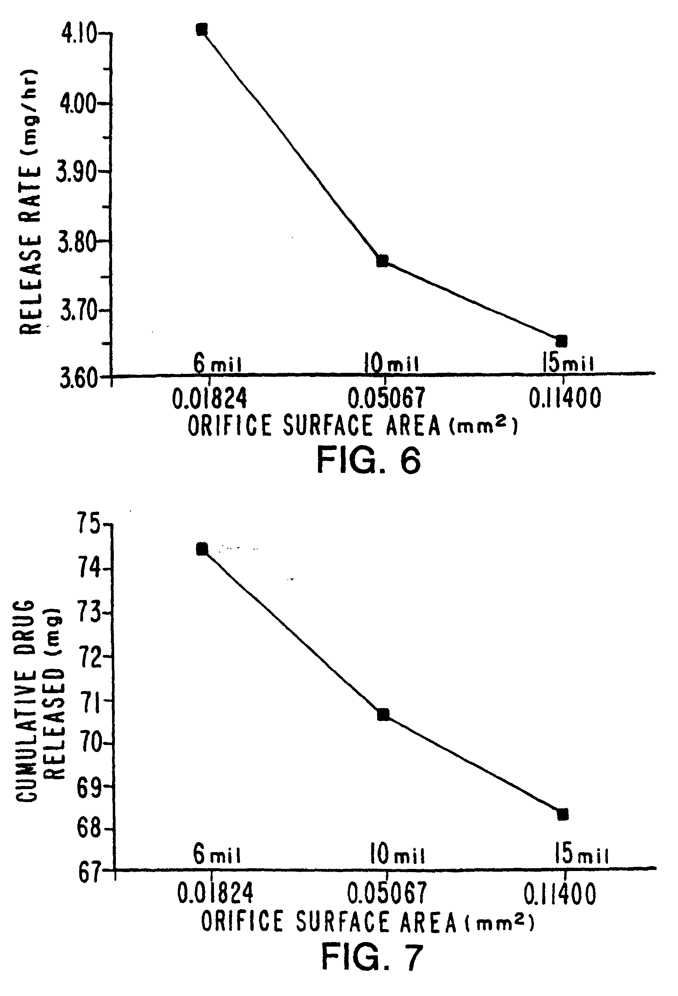

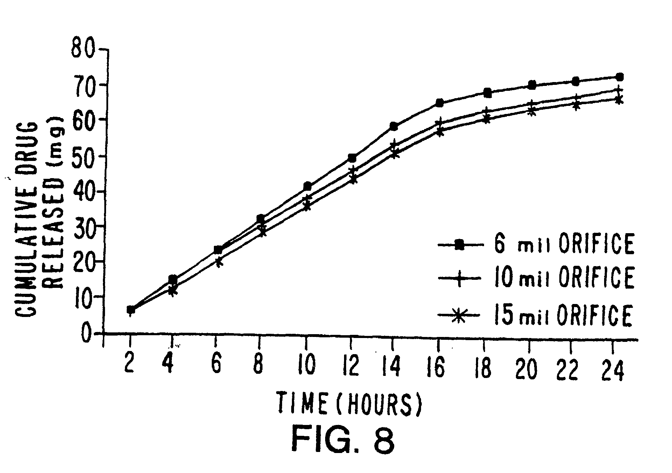

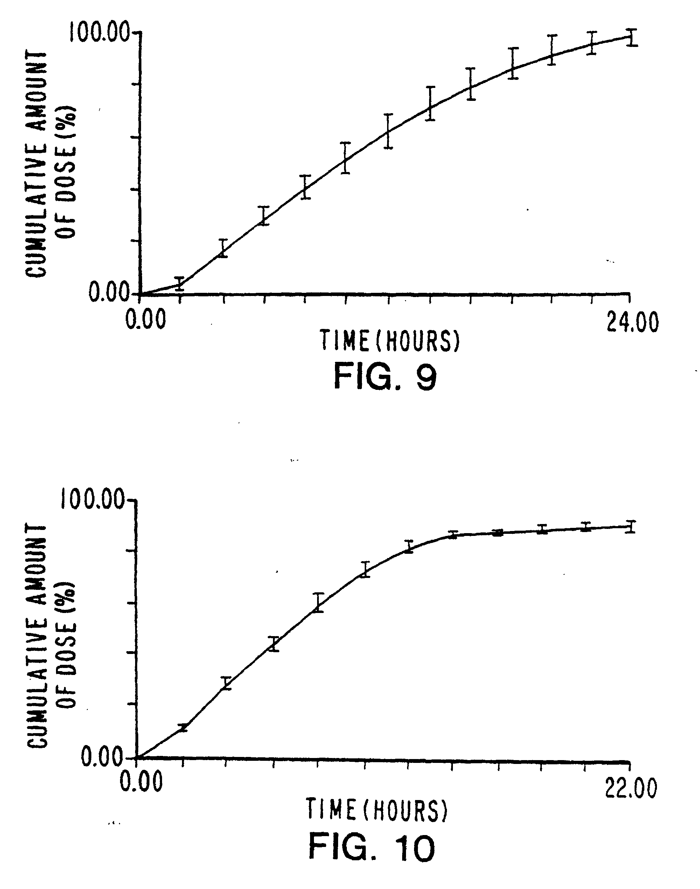

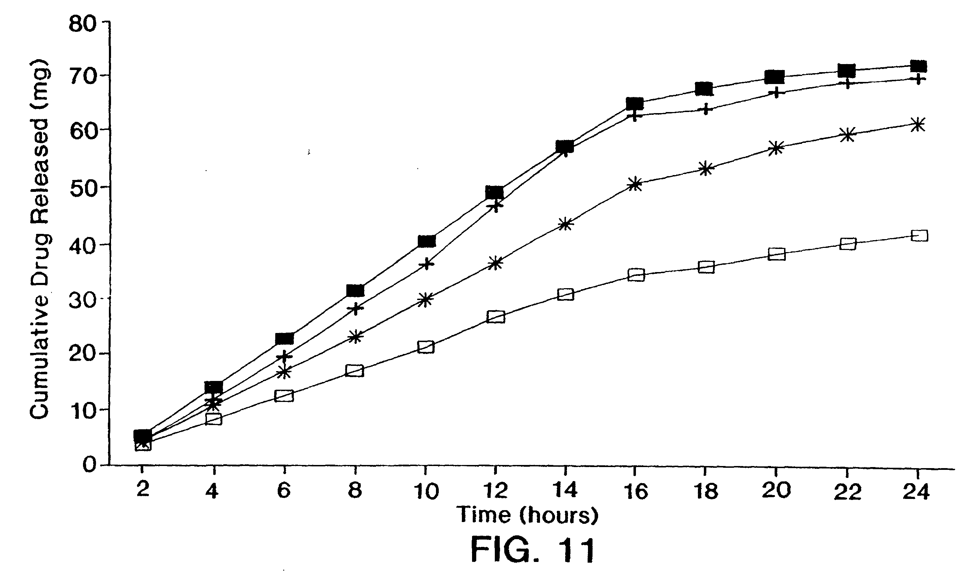

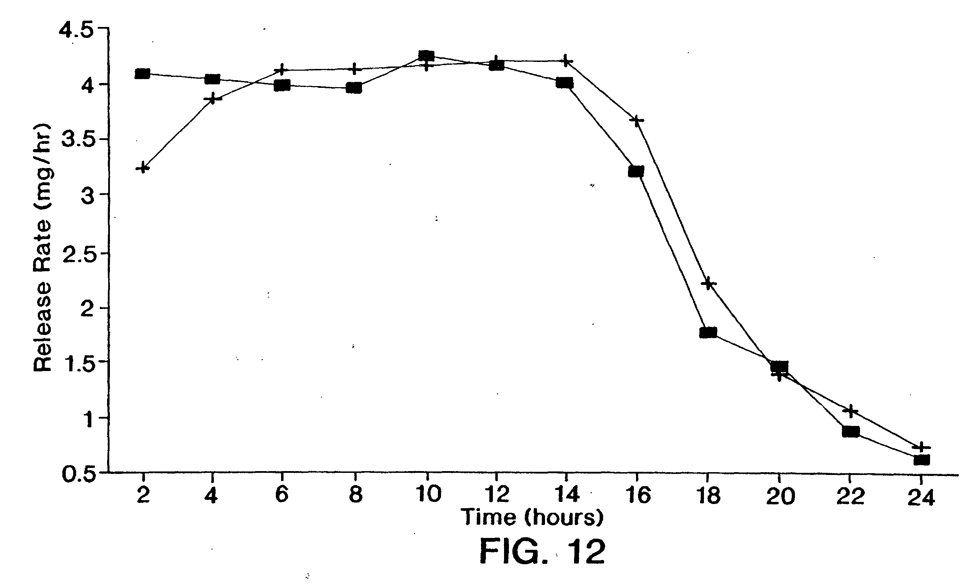

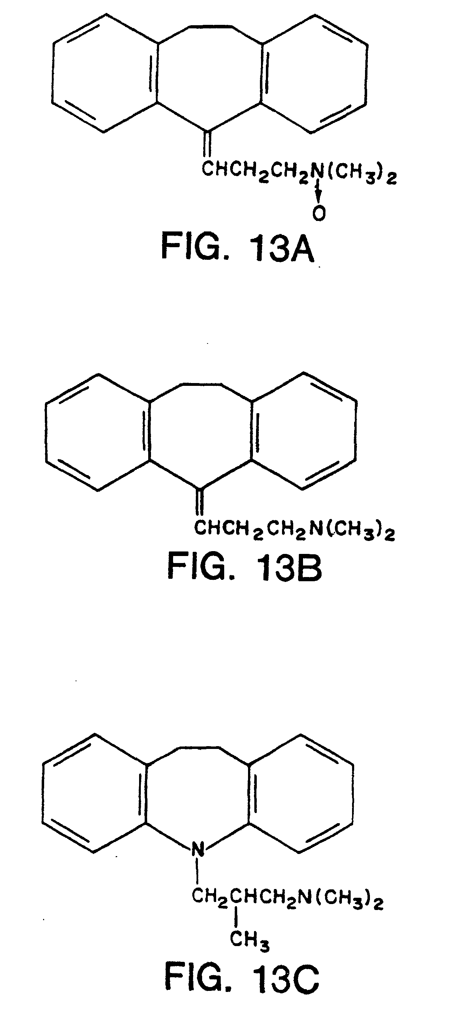

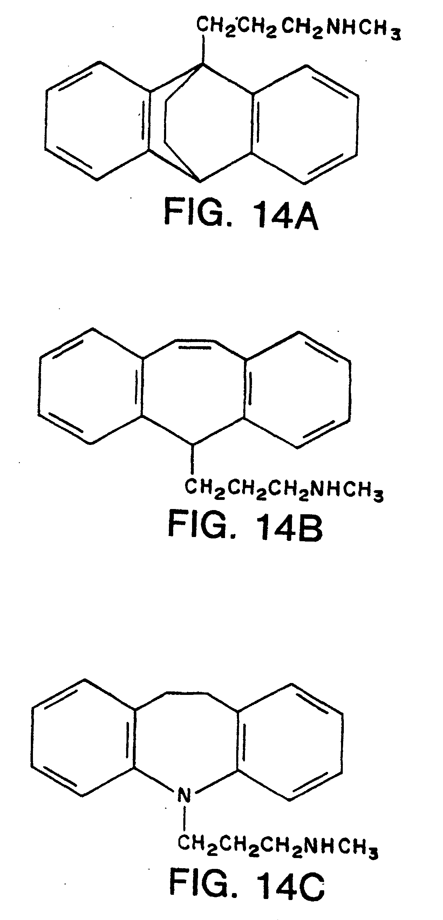

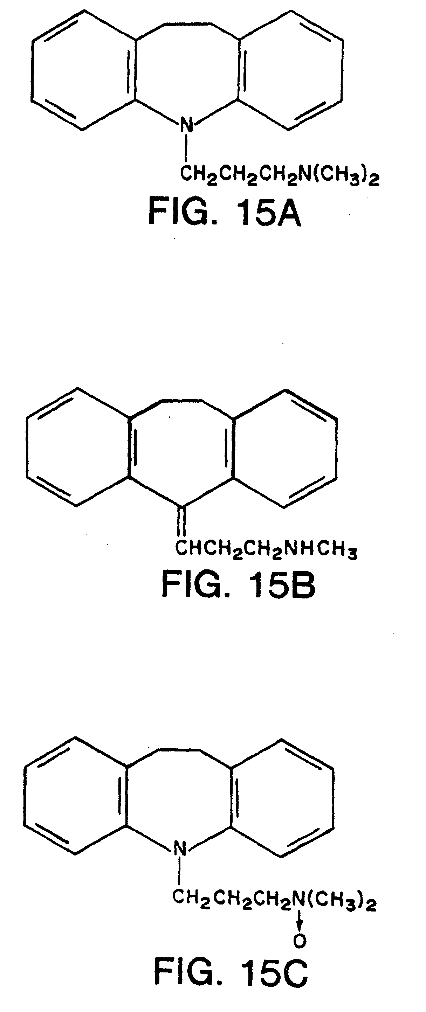

In the drawing figures, which are not drawn to scale, but are set forth to illustrate various embodiments of the invention, the drawing figures are as follows:
Drawing Figure 1 is a closed view of a dosage form designed and shaped for administering orally the dosage form comprising a antidepressant tricyclic drug for pharmacological antidepressant therapy:
Drawing Figure 2 is an opened-view of drawing Figure 1 for depicting a manufacture comprising a dosage form that houses an antidepressant composition for administering to a patient;
Drawing Figure 3 is an opened-view of the dosage form of drawing Figure 1, illustrating an antidepressant composition and a push composition initially in layered arrangement in the dosage form;
Drawing Figure 4 is a view illustrating a dose of tricyclic antidepressant on the exterior surface of the wall of the dosage form;
Drawing Figure 5 is a view illustrating a dosage form manufactured with a plurality of drug release rate governing pores;
Drawing Figure 6 is a graph illustrating the release rate vs. the orifice surface area;
Drawing Figure 7 is a graph that depicts the cumulative drug released vs. the orifice surface area;
Drawing Figure 8 is a graph that depicts the cumulative drug released in mg in an artificial intestinal fluid;
Drawing Figure 9 is a graph that depicts the release of drug over a pH range of 7 to 11;
Drawing Figure 10 is a graph that depicts the cumulative amount of amitriptyline relieved in 24 hours;
Drawing Figure 11 is a graph depicting the cumulative amount of amitriptyline released in 24 hours;
Drawing Figure 12 is a graph that depicts the drug release in milligrams over 24 hours measured by different tests; and
Drawing Figure 13, 14 and 15 depict antidepressant drugs that can be administered by the method of the invention.

In the drawing figures and in the specification, like parts in related figures are identified by like numbers. The terms appearing earlier in the specification and in the description of the drawing figures, as well as in embodiments thereof, are further described elsewhere in the disclosure.

### DETAILED DESCRIPTION OF THE DRAWING FIGURES

Turning now to the drawing figures in detail, which drawing figures are in example of the dosage forms and compositions provided by this invention, and which examples are not to be construed as limiting the invention, one example of the dosage form is illustrated in drawing Figure 1 and designated by the numeral 10. In drawing Figure 1, a sustained-release dosage form 10 is depicted that comprises a body member 11 comprising a wall 12 that surrounds and encloses an internal compartment , not seen in drawing Figure 1. Wall 12 keeps its physical integrity and structure in the presence of osmotic and hydrodynamic pressure generated within dosage form 10 during operation of dosage form 10 in a biological environment of use. Dosage form 10 comprises at least one exit means 13 for connecting the interior of dosage form 10 with the exterior environment of use.

In drawing Figure 2, dosage form 10, is seen as a sustained-release dosage form manufactured as a tablet, seen in opened-view. In drawing Figure 2, dosage form 10 comprises a body 11, wall 12, that is sectioned at 14 for depicting wall 12 that surrounds and forms internal compartment 15. Wall 12 comprises at least one exit means 13 that connects compartment 15 with the exterior of dosage form 10. Dosage form 10 can comprise more than one exit means 13.

Wall 12 of dosage form 10 comprises a composition that is permeable to the passage of an exterior fluid present in the fluid environment of use, and the wall-forming composition is substantially impermeable to the passage of an tricyclic antidepressant drug, and to other nonfluid components present in compartment 15. The composition maintains its physical and chemical integrity, that is, it does not change its chemical nature during the drug delivery period, independently of the structure of wall 12. Wall 12 comprises from 40 weight percent, (wt%), to 100 wt% of a cellulose wall-forming polymer. The polymer comprises a member selected from the group consisting of cellulose acylate, cellulose diacylate, cellulose triacylate, cellulose acetate, cellulose diacetate, and cellulose triacetate. In another, operative manufactured, wall 12 comprises additionally to the cellulose acylates from 0 wt% to 20 wt% of a different cellulose ether selected from the group consisting of hydroxypropylcellulose, hydroxypropylethylcellulose, hydroxybutylcellulose and hydroxypropylmethylcellulose; and from 0 wt% to 20 wt% of a polyethylene glycol. The total weight percent of all components comprising wall 12 is equal to 100 wt%.

Representative of wall-forming semipermeable polymers that are not metabolized in the gastrointestinal tract, which are excreted intact, and which are substituted by one to three acetyl groups, or by one or two acetyl groups and a further acyl radical other than acetyl groups, comprises cellulose agar acetate, cellulose acetate ethyl carbonate, cellulose acetate succinate, cellulose acetate dimethylaminoacetate, cellulose acetate chloroacetate, cellulose acetate ethyl oxalate, cellulose acetate methyl sulfonate, cellulose acetate butyl sulfonate, cellulose acetate propionate, cellulose acetate dimethylaminoacetate, cellulose acetate bulytate, cellulose acetate octate, cellulose acetate laurate, cellulose acetate p-toluenesulfonate, cellulose acetate butyrate, and cellulose acetate valerate. Other wall-forming composition comprise semipermeable copolymers of aikalene oxides, and alkyl glycidyl ethers, and mixtures of semipermeable water-insoluble acrylates, such as the copolymer of ethyl acrylate and methyl methacrylate.

Dosage form 10, as seen in Figure 2, in compartment 15, comprises a tricyclic antidepressant drug 16, represented by dots. The antidepressant drug 16 comprises 2 wt% to 99 wt% of an antidepressant drug 16 selected from the group consisting of amitriptylinoxide; amitriptyline, trimipramine, maprotiline, protriptyline, desipramine, imipramine, nortriptyline and imipramine oxide, their therapeutically acceptable salts, the base, the racemate and the geometric isomers. The therapeutic dose of the tricyclic antidepressants can be administered once a day, or more than once a day, in daily doses of 1 mg to 750 mg per dosage form 10. Individual dosage form 10 can comprise 1, 5, 15, 20, 25, 30, 50, 75, 100, 125, 140, 150, 175, 200, 220, 250, 300, 450, 500 and 650 mg of the tricyclic antidepressant drug 16. The pharmaceutically acceptable non-toxic salts useful for dispensing the tricyclic antidepressants comprise a member selected from the group consisting of therapeutically acceptable salts including inorganic, organic, acetic, maleic, hydrochloric, hydrobromic, sulfuric, phosphoric, propionic, butyric, tartaric, citric, fumaric, lysine, succinic, palmitic, lactic, embonic, ascorbic, pamoatic, hydrobromic, methanesulfonic, glycine, and salts.

Compartment 15 contains a pharmaceutically acceptable polymeric carrier 9 represented by v. The pharmaceutically acceptable polymers for the purpose of this invention include osmopolymers, hydrogels, and osmogels, which are equivalents for the present purpose. The pharmaceutically acceptable polymers 9 comprise a member selected from the group consisting of a polyalkylene oxide possessing a 50,000 to 900,000 weight-average molecular weight and a carboxyalkylcellulose possessing a 15,000 to 175,000 weight-average molecular weight. Representative of polyalkylene oxides include polymethylene oxide of 50,000 molecular weight, polyethylene oxide of 100,000 molecular weight, polyethylene oxide of 200,000 molecular weight, polyethylene oxide of 300,000 molecular weight, polypropylene oxide of 400,000 molecular weight, and a copolypropylene oxide-polyethylene oxide of 600,000 molecular weight. The polyalkylene oxide used as pharmaceutically acceptable carriers exclude polyalkylene glycols. Representative of carboxyalkylcellulose comprise a member selected from the group consisting of carboxyalkylcellulose, alkali carboxyalkylcellulose, sodium carboxymethylcellulose, potassium carboxymethylcellulose, alkali carboxyethylcellulose, carboxyalkylhydroxyalkylcellulose, carboxymethylhydroxyethylcellulose, carboxyethylhydroxyethyl-cellulose, and carboxymethylhydroxypropylcellulose. The osmopolymer exhibit a concentration gradient across wall 12 resulting in fluid imbibition across wall 12 into compartment 15. The imbibed fluid enables osmopolymer 9 to perform as a pharmaceutically-acceptable carrier for dispensing the antidepressant drug 16 to a human patient in need of therapy.

The antidepressant composition comprises a binder 8 represented by slanted dashes. The binders for the purpose of this invention include acacia, starch, gelatin, polyvinylpyrrolidone, copolymers of polyvinylpyrrolidone and vinyl alcohol, copolymers of polyvinylpyrrolidone with vinyl chloride, copolymers of polyvinylpyrrolidone with vinyl butyrate, copolymers of polyvinylpyrrolidone with vinyl laurate, and copolymers of polyvinylpyrrolidone with vinyl stearate. The binder serves to bind the therapeutic composition during manufacture and the binders are used in from 0.01 to 10 mg. The polyvinylpyrrofidone possesses a 5,000 to 175,000 viscocity-average molecular weight.

The therapeutic composition comprises a lubricant 7 represented by a hexagon. The lubricant is used during manufacture to prevent sticking to die walls or punch faces. Typical lubricants include magnesium stearate, sodium stearate, stearic acid, calcium stearate, magnesium oleate, oleic acid, potassium oleate, sodium oleate, caprylic acid, sodium stearyl fumarate, and magnesium palmitate. The amount of lubricant present in the therapeutic composition is 0.00 mg to 10 mg.

The therapeutic antidepressant composition comprises an osmagent 6 represented by an opened-square. The osmagents are known also as osmotic agents, osmotlcally effective compounds, and osmotic solutes. They exhibit an osmotic pressure gradient across semipermeable wall 12 and they produce 2.5 to 500 atmospheres of pressure. The osmagents 6 imbibe aqueous fluid through wall 12 for hydro-osmotically delivering the antidepressant drug 16 to a patient. Representative osmagents are a member selected from the group consisting of magnesium sulfate, magnesium chloride, sodium chloride, potassium chloride, potassium sulfate, sodium sulfate, mannitol, sorbitol, glucotrol, urea, sucrose, fructose, lactose, glucose-fructose blend, and urea-sodium chloride blend. The amount of osmagent in the therapeutic, antidepressant drug composition is 2 mg to 125 mg.

In drawing Figure 3 dosage form 10, manufactured as an osmotic dosage form, is seen in opened view. In drawing Figure 3, dosage form 10 comprises body 11, wall 12 that is sectioned at 14 for depicting wall 12 that surrounds and defines an internal compartment 15. Wall 12 comprises at least one exit means 13 that connects compartment 15 with the exterior of dosage form 10. Dosage form 10 can comprise more than one exit means 13.

Dosage form 10 comprises an antidepressant drug 16 composition 5. Antidepressant drug composition 5 is present initially as a layer. Drug 16 is encased and surrounded with a formulation dispensing agent 19 that serves to ensure antidepressant drug 16 is dispersed in a dispensable formulation to effect the administration of the intended therapeutic dose of antidepressant drug 16 from dosage form 10. Also drug 16 is encased to lessen the incidence of stickiness of the tricyclic antidepressant drug. Representative of formulating dispensing agent 19 for surrounding drug 16 comprises a watersoluble polymer, such as cyclic poly-N-vinyl amide, and a member selected from the group consisting of poly-N-vinylmethylacetamide, poly-N-vinylethylacetamide, poly-N-vinylmethylpropionamide, poly-N-vinylethylpropionamide, poly-N-vinylmethyl-isobutyramide, poly-N-vinyl-2-pyrrolidone, poly-N-vinyl-2-piperidone also known as polyvinyl pyrrolidone and as poly-n-vinyl-pyrrolidone, and poly-N-vinyl-3-methyl-2-pyrrolidone, having a 3,000 to 750,000 molecular weight. In another manufactured drug 16 is microencased or surrounded with a hydroxyalkylcellulose or a hydroxypropylalkylcellulose, wherein alkyl is 1 to 6 carbons selected from the group consisting of methyl, ethyl, propyl, butyl, pentyl and hexyl, which cellulose ether polymers comprise a 9,000 to 1,750,000 molecular weight, the polymers are exemplified by hydroxymethylcellulose, hydroxypropylcellulose, hydroxypropylmethylcellulose, hydroxypropylethylcellulose, hydroxypropylbutylcellulose, and hydroxypropylhexylcellulose. Antidepressant drug 16 surrounded by dispensing agent 19, additionally may comprise in another manufacture a dispensing agent 19 comprising a swellable polyvinyl acetate having a 3,000 to 750,000 molecular weight, or a polyvinyl alcohol having a 250 to 4,500 degree of polymerization.

The present invention in one presently preferred embodiment is characterized by overcoming the tribulations associated with the tricyclic antidepressant drug 16 by granulating antidepressant drug 16 with formulating agent 19 for improved manufacturing and improved therapy. The granulation of tricyclic antidepressant drug 16 by the invention improves the flow properties of tricyclic antidepressant drug 16. The tricyclic antidepressant drugs and derivatives thereof generally are sticky and granulation of these drugs by coating drug 16 with dispensing formulation agent 19, as disclosed above in this specification, substantially prevents and substantially lessens adhesion of drug 16 to manufacturing machinery, and the granulation enhances the dispersion of the antidepressant drug 16 in the gastrointestinal tract, which can lead to improved therapy.

The expression granulation as used for the purpose of this invention denotes coated granules of antidepressant, tricyclic drug 16 with formulating dispensing agent 19. The granules exhibit a diameter of 0.01 mm to 1.75 mm and the coated drug 16 granules are released from dosage form 10 independent of the acid-base environment of the gastrointestinal tract. The mean coating thickness of dispensing agent 19 is in the range of 10 to 100 microns (0.001 mm 0.40 mm) and comprises 0.01 mg to 40 mg of the coating formulation 19 around drug 16. The pharmaceutical coated granules of antidepressant tricyclic drugs comprise different shapes and sizes for example, uniform-edged shapes, spheroidal, oval, round, bean, and other shapes. A more preferred even shape distribution enables the coating to be of a more uniform thickness. The pharmaceutical coated granules of drug 18 are capable of presenting antidepressant drug 16 in an absorbable form to the absorbing surface of the gastrointestinal tract. The coated antidepressant drug granules are characterized by surrounding antidepressant drug granules with a formulating agent 19 designed for oral therapy as the drug is delivered throughout the gastrointestinal tract in a transit time, in humans about 1 hour to 30 hours. The coated antidepressant drug 16 is delivered at a drug linear release rate from the beginning of the release period to the end of the drug delivery period.

Drug layer 5 comprises additionally a polymer 20 depicted by a straight line, for transporting antidepressant coated drug 16 from dosage form 10. Polymer 20, in one manufacture, comprises the structure (0-CH₂-CH₂)ₙ, wherein n is a positive whole number of 2,000 to 20,000. Representative of the polymer 20 is a poly(oxyethylene) of average molecular weight of 1 x 10⁵, 2 x 10⁵, 3 x 10⁵, to 8 x 10⁵. Drug layer 5, comprises in another manufacture a polymer composition comprising a first polymer and a second polymer blended together to function as a unit polymer 20 comprising coated drug 16. The first polymer comprises the polymer described immediately above, and of the second polymer of the structure (0-CH₂-CH₂)ₙ, wherein n is a whole number of 6,500 to 7,500 as represented by poly(oxyethylene) of the approximate average molecular weight of 1 x 10⁵ to 3 x 10⁵. In the polymer composition, the concentration of the first polymer is 10 to 60 wt% and in one preferred range, expressed in milligrams of 0.5 mg to 75 mg and the composition of the second polymer is 1 to 65 wt%, and in one preferred embodiment, expressed as mg from 1.5 mg to 75 mg. The concentration of the first and second polymer, when presented together in dosage form 10, is 5 to 80 wt%. Polymer 20, present as a single polymer, or as a first and second polymer pair, effectively transports, in both manufactures, antidepressant drug 16 from dosage form 10, and they release antidepressant drug 16 to a drug receptor for antidepressant therapy. It is unexpected polymer 20 can perform its housing-transporting-releasing-compositional functions, as a complex drug composition comprising the antidepressant drug 16, essentially-free of any bonding of drug 16 to polymer 20 and free of antidepressant precipitation in compositional layer 5. Drug layer 5 comprises optionally from 0 wt% to 3 wt% of a lubricant 7 such as magnesium stearate or calcium stearate; from 0 to 20 wt% of an osmotically active compound 22 such as a member selected from the group consisting of an inorganic salt, an organic salt, a compound containing an amino group, a carbohydrate, an osmotic acid and an osmotic ester; and 0 wt% to 4 wt% of an anti-oxidant 23 for imparting stability to the antidepressant drug 16 composition, said anti-oxidant comprising a member selected from the group consisting of ascorbic acid, 2,3-butyl hydroxyanisole, mon-tertiary butylated hydroquinone, and butylated hydroxytoluene. The total weight percent of all components in composition 5 is equal to 100 wt%.

Second composition layer 17 comprises 35 wt% to 80 wt% of a polymer 24 comprising the repeating molecular unit (0-CH₂-CH₂)ₙ, wherein n is a positive whole number of 22,000 to 230,000. Representative of polymer 24, comprising the repeating molecular unit, is a poly(alkalene oxide) comprising poly(ethylene oxide) of an approximate average molecular weight of 1 x 10⁶ to 10 x 10⁶, and in another embodiment alkali carboxymethylcellulose such as sodium carboxymethylcellulose having a 75,000 to 1,500,000 molecular weight.

Polymer 24 provides unexpected operating advantages as the polymer maintains its chemical composition during operation as it imbibes an external aqueous fluid including biological fluid through wall 12 while simultaneously expanding and pushing antidepressant drug composition 5 from dosage form 10, essentially-free of substantially mixing with push-expandable layer 17. Second layer 17 comprises also 0.5 wt% to 35 wt% of an osmotically active compound 25, represented by small squares. Representation of osmotically effective compounds 25 comprises salts, osmotic esters, carbohydrates, and osmotic acids, such as a member selected from the group consisting of magnesium sulfate, magnesium chloride, sodium chloride, lithium chloride, potassium sulfate, sodium sulfate, lithium sulfate, potassium chloride, ammonium chloride, potassium lactate, mannitol, urea, inositol, magnesium succinate, tartaric acid, ribose, raffinose, sorbitol, sucrose, fructose, glycose, glucose, arabinose, galactose, dihydrogen phosphate, sodium acetate, potassium acetate, magnesium succinate, sodium benzoate, sodium citrate, sodium ascorbate, glycine, leucine, alanine, urea, and methionine. Second layer 17 comprises optimally 0.0 wt% to 20 wt% of a cellulose ether 24 represented by small triangles 26. Representative of cellulose ethers 24 comprises a member selected from the group consisting of hydroxypropylcellulose, hydoxypropylethylcellulose, hydroxyalkylethylcellulose, hydroxybutylcellulose, hydroxypentylcellulose, and hydroxypropylmethylcellulose having a molecular weight of 9,000 to 162,500. The composition comprises 0 wt% to 5 wt% of a lubricant such as stearic acid, magnesium stearate, calcium stearate, calcium oleate, oleic acid, and caprylic acid. The total weight of all composition layer 17 ingredients is equal to 100 wt%. The polymers disclosed herein are commercially available from the Union Carbide Corporation, South Charleston, West Virginia.

Drawing Figure 4 illustrates another dosage form 10 provided by the invention. Drawing Figure 4 depicts a dosage form 10 comprising body 11, wall 12, exit means 13, and the further inventive embodiment, a composition 27 comprising a tricyclic antidepressant drug 16 on the exterior surface of wall 12. Exterior composition 27 comprises a dosage unit amount of antidepressant drug 16 for an initial pulsed dose of drug 16 to the environment of use, the gastrointestinal tract of a warm-blooded animal including humans. The initial pulse is the first dose of tricyclic antidepressant drug 16. Exterior composition 27 comprises from about 0.1 to 99.9 wt% of drug 16, and from 99.9 to 0.1 wt% of a pharmaceutically acceptable carrier for drug 16, with the total weight percent of all composition 27 forming members equal to 100%. In a more preferred embodiment the initial pulse dose of tricyclic antidepressant drug 16 is from 10 to 80 wt% and from 90 to 20 wt% carrier. The carrier is a means for coating drug 16 onto the exterior surface of wall12. In the fluid environment of use, the carrier releases drug 16 thereby providing the initial dose of drug 16 to the environment of use. The carrier releases the initial dose in from greater than zero time up to 1 hour, and in one manufacture a pulsed dose time of from several minutes, (about 3 minutes) up to 30 minutes. Typical pharmaceutically acceptable carrier means include a hydrophilic polymer, that is in a presently preferred embodiment a member selected from the group consisting of hydroxymethylcellulose, hydroxyethylcellulose, hydroxypropylcellulose, hydroxyalkycellulose, hydrobutylcellulose, hydroxypropylmethylcellulose, and hydroxypropylethylcellulose having a 9,000 to 262,500 molecular weight.

Drawing Figure 5 depicts another dosage form provided by the invention. In drawing Figure 5, and in the present invention, the expression, "exit means 13", as used herein, comprises means and methods suitable for the metered release of the therapeutic drug 16 from compartment 15 of dosage form 10. The exit means 13 comprises at least one passageway, orifice, bore, aperture, pore, porous element, hollow fiber, capillary tube, porous overlay, porous insert, and porous element that provides for the osmotic controlled release of coated, antidepressant tricyclic drug 16. The expression "exit means 13" includes a material that dissolves, erodes, or is leached from wall 12 in a fluid environment of use to produce at least one osmotic dimensional passageway pore 13 in dosage form 10. Drawing Figure 5 depicts wall 12 comprising a plurality of exit pores 13. Exit pores 13 can be formed by adding a porosigin to wall 12 that will function to provide an exit pore 13. Representation of porosigins or pore-forming materials suitable for forming a passageway, or a multiplicity of passageways, comprises a leachable poly(glycolic) acid, or poly(lactic) acid polymer in wall 12, a gelatinous filament, poly(vinyl alcohol), leachable polysaccharides, salts, and oxides. Typical salts include water soluble inorganic salts such as sodium chloride, potassium chloride, and potassium phosphate. A pore passageway, or more than one pore passageways can be formed by leaching a leachable compound, such as sorbitol, glucose, lactose, succinic acid, and sodium succinate from wall 12, The porosigin pore former is pharmaceutically acceptable thereby providing the acceptability of dosage form 10 in an animal environment of use. Exit means 13 can be provided by gas generation in wall 12, by laser drilling, by mechanical drilling, by sonic drilling, by electric discharge, and by etched nuclear tracking procedures. The exit passageway 13 can have different shapes, such as round, triangular, egg, square, oval, elliptical, for the metered release of drug 16 from dosage form 10. Dosage form 10 can be construed with one or more passageways 13 in spaced apart relationship on a single surface or on more than one surface of dosage form 10. Passageways and equipments for forming passageways are disclosed in U.S. Patent Nos. 3,845,770; 3,916,899; 4,063,064; 4,088,864 and 4,816,263. Passageways formed by leaching are disclosed in U.S. Patent Nos. 4,200,098 and 4,285,987.

Dosage form 10 controls the exit 13 surface area for releasing drug 16 essentially independent of the pH of the drug receiving biological receptor, thereby essentially preventing crystallizing drug 16 in exit 13, or essentially preventing blocking exit 13 with nonreleased drug 16. For example, the antidepressant drug 16 amitriptyline hydrochloride has a pKa of 9.4, and it becomes increasingly insoluble at a higher pH. The drug at 7.5 pH, the pH of artificial intestinal fluid, is soluble, but the solubility is less compared to the drug solubility in artificial gastric fluid at pH 6. The drug amitriptyline hydrochloride solubility is more than 600 mg/ml an artificial gastric fluid. In artificial intestinal fluid at 37°C the drug dissolution rate in artificial intestinal fluid at pH 7.5 is low compared to the dissolution rate in a lower pH such as gastric fluid having a pH of about 2. That is, it takes longer to dissolve an equal amount of amitriptyline hydrochloride in artificial intestinal fluid than in artificial gastric fluid. The dissolution rate is sensitive to pH changes, and when drug 16 is pumped from the dosage form it tends to crystallize at higher pH in orifice exit 13, or to partially block orifice exit 13, which in either instance, slows the drug release rate, and decreases the cumulative amount of drug released from the dosage form. The present invention by providing coated granules of antidepressant tricyclic drug 16, and by providing an orifice in the range of 1 mil to 30 mil (0.0254 mm to 0.760 mm), essentially overcomes the problems discussed above. In accompanying drawing Figure 6, the release rate for noncoated amitriptyline hydrochloride is compared with the orifice surface area of the dosage form. In drawing Figure 6, the release rate is given on the y-axis and the orifice surface area in mils and in mm² is given on the x-axis. The release of drug from the dosage form was measured in artificial intestinal fluid. The results of Figure 6 show the release rate decreases for a noncoated drug even with an increase in orifice size. Drawing Figure 7 depicts the cumulative drug release for tricyclic amitriptyline hydrochloride in mg, plotted against the orifice surface area in mm² for an orifice of 6 mil to 15 mil in the artificial intestinal fluid which accompanies Figure 6. Drawing Figure 8, measured in artificial intestinal fluid at pH 7, plots the cumulative drug release over a time of 24 hours for three orifices, 6 mil (0.152 mm), 10 mil (0.254 mm), and 15 mil (0.381 mm). Drawing Figure 9 depicts the cumulative amount of drug release in mg, over a period of 24 hours for a coated drug through a 10 mil (0.254 mm) exit orifice illustrates about 100% of the drug is delivered free of crystalizing in the orifice. Drawing Figure 10 depicts the release rate measured in artificial gastric fluid over zero to two hours and then in artificial intestinal fluid over two to twenty-four hours, through a 10 mil (0.254 mm) orifice illustrates about 90% of the drug is delivered under these conditions. The scientific studies reported herein demonstrate the unexpected results obtained by the novel and unique composition and coated drug by this invention. The antidepressant drug is dose delivered independent of pH as found in the gastrointestinal drug and free from sticking or blocked delivery to a patient.

### DESCRIPTION OF PROCESSES FOR MANUFACTURING THE DOSAGE FORM OF THE INVENTION

Wall 12 of osmotic dosage form 10 can be formed in one technique using the air suspension procedure. This procedure consists in suspending and tumbling the compressed compositions in a current of air and wall-forming composition until a wall is applied to the drug-forming compartment. The air suspension procedure is well-suited for independently forming the wall. The air suspension procedure is described in U.S. Patent No. 2,799,241; J. Am. Pharm. Assoc., Vol. 48, pp 451 to 454, 1959; and ibid. Vol. 49, pp 82 to 84, 1960. Osmotic dosage forms can also be coated with a wall-forming composition in a Wurster® air suspension coater, using an acetone-water cosolvent, for example, 90:10, wt:wt, using 2,5 to 7 wt% polymer solids. The Aeromatic® air suspension coater, using a methylene dichloride methanol cosolvent, for example, 87:13, v:v, also can be used for applying the wall. Other wall-forming techniques, such as pan coating, can be used for providing the dosage form. In the pan coating system, wall-forming compositions are deposited by successive spraying of the composition on the bilayered compartment, accompanying by tumbling in a rotating pan. A larger volume of cosolvent can be used to reduce the concentration of polymer solids to produce a thinner wall. Finally, the wall coated compartments are laser or mechanically drilled and dried in a humidity oven at 40°C to 50°C/50RH, wherein RH is relative humidity, for a week to free the dosage form of solvent. Generally, the walls formed by these techniques have a thickness of 2 to 20 mils (0.051 to 0.51 mm) with a presently preferred thickness of 2 to 6 mils (0.051 to 0.15 mm).

Dosage form 10 of the invention is manufactured by standard manufacturing techniques. For example, in one manufacture, the beneficial antidepressant drug and other ingredients comprising the first composition facing the exit means are blended and pressed into a solid layer. The drug and other ingredients can be blended also with a solvent and mixed into a solid or semisolid formed by conventional methods such as ball-milling, calendaring, stirring, blending or rollmilling and then pressed into a preselected orally administrable shape. The composition possesses dimensions that correspond to the internal dimensions of the area the compositional layer is to occupy in the dosage form and it also possesses dimensions corresponding to the second compositional layer for forming a contacting arrangement therewith. Next, the osmopolymer or second layer is placed in contact with the drug or first layer. The layering of the drug first compositional layer and the osmopolymer second compositional layer can be fabricated by conventional press-layering techniques. Finally, the two compositional layer compartment-forming members are surrounded and coated with an outer semipermeable wall. A passageway is laser drilled through the wall to contact the drug layer, with the dosage form optically oriented automatically by the laser equipment for forming the passageway on the preselected surface.

In another manufacture, dosage form 10 is manufactured by the wet granulation technique. In the wet granulation technique, the drug and the other ingredients comprising the first drug layer are blended using an organic or inorganic solvent, such as, isopropyl alcohol-methylene dichloride 80:20 v:v as the granulation fluid. Other granulating fluid, such as water or denatured alcohol 100%, can be used for this purpose. The ingredients forming the first drug layer are individually passed through a 40 mesh screen and then thoroughly blended in a mixer. Next, other ingredients comprising the first drug layer are dissolved in a portion of the granulation fluid, such as the cosolvent described above. Then, the latter prepared wet blend is slowly added to the drug blend with continual mixing in the blender. The granulating fluid is added until a wet blend is produced, which wet mass then is forced through a 20 mesh screen onto oven trays. The blend is dried for 12 to 30 hours at 25°C to 50°C. The dry granules are screened then with a 16 mesh screen. Next, a lubricant is passed through an 60 mesh screen and added to the dry screened granule blend. The granulation is put into milling jars and mixed on a jar mill for 2 to 10 minutes. The first and second layer compositions are pressed into a layered tablet, for example, in a Manesty® layer press.

Another manufacturing process that can be used for providing the compartment-forming composition comprises blending the powdered ingredients in a fluid bed granulator. After the powdered ingredients are dry blended in the granulator, a granulating fluid, for example, poly(vinylpyrrolidone) in water, is sprayed onto the powders. The coated powders are then dried in a granulator. This process coats-agglomerates all the ingredients present therein while spraying the granulating fluid. After the granules are dried, a lubricant such as stearic acid or magnesium stearate is blended as above into the mixture. The granules are pressed then in the manner described above.

The controlled-delivery device of this invention is manufactured in another embodiment by mixing a drug with composition-forming ingredients and pressing the composition into a solid layer possessing dimensions that correspond to the internal dimensions of the compartment space adjacent to a passageway. In another embodiment, the drug and other first composition-forming ingredients and a solvent are mixed into a solid, or a semi-solid, by conventional methods such as ballmilling, calendaring, stirring or rollmilling, and then pressed into a preselected layer-forming shape.

In the manufactures as presented above, the manufacture comprising a layer of a composition comprising an osmopolymer and an optional osmagent is placed in contact with the layer comprising the drug, and the two lamina comprising the layers are surrounded with a semipermeable wall. The layering of the first drug composition and the second osmopolymer compressing an optional osmagent composition can be accomplished by using a conventional two-layer tablet press technique. The wall can be applied by techniques such as molding, spraying or dipping a pressed shape comprising wall-forming materials. Another and presently preferred technique that can be used for applying the wall in the air suspension coating procedure. This procedure consists in suspending and tumbling the two layers in a current of air until the wall-forming composition surrounds the layers. The air suspension procedure is described in U.S. Patent No. 2,799,241; J. Am. Pharm. Assoc., Vol. 48, pp 451-454 (1959); and, ibid, Vol. 49, pp 82-84 (1960). Other standard manufacturing procedures are described in Modern Plastics Encyclopedia. Vol. 46, pp 62-70 (1969); and in Pharmaceutical Science, by Remington, 14th Ed., pp 1626-1648 (1970), published by Mack Publishing Co., Easton, Pennsylvania. A coating can be applied also by a rotor granulator. One example of a rotor granulator is the Vector Freund® Spir-A-Flow granulator, and another example is the Glatt® rotor granulator. In general, a rotor granulator comprises a pressing chamber with a rotor at its lower portion. Air is introduced at the level of the rotor for fluidization of the product bed. This air may enter the chamber through the opening between the rotor and the stator and/or through a second opening about midway across the radius of the rotor. The introduction of air results in a spiral and twisting air pattern within the chamber. When the inert starting seeds are introduced into the chamber, the combination of rotor and air circulation pattern is purported to provide higher individual particle densities, and rounder and smoother granules. Once the starting seeds have been fluidized in the rotor granulator, the solution is introduced through the spray guns to spray the granules, to yield coated granules. A rotor granulator is known in U.S. Patent No. 4,925,675. Another coating machine for the present purpose is in Accelacota® Coater manufactured by Manesty Co.

Exemplary solvents suitable for manufacturing the wall, the laminates and laminae include inert inorganic and organic solvents that do not adversely harm the materials and the final wall of the delivery device. The solvents broadly include members selected from the group consisting of aqueous solvents, alcohols; ketones, esters, ethers, aliphatic hydrocarbons, halogenated solvents, cycloaliphatics, aromatics, heterocyclic solvents, and mixtures thereof. Typical solvents include acetone, diacetone, alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monoethylacetate, methylene dichloride, ethylene dichloride, propylene dichloride, carbon chloroform, nitroethane, nitropropane, tetrachloroethane, ethyl ether, isopropyl ether, cyclohexane, cycloctane, toluene, naphtha, 1,4-dioxane, tetrahydrofuran, diglyme, aqueous and nonaqueous mixtures thereof, such as acetone and water, acetone and methanol, acetone and ethyl alcohol, methylene dichloride and methanol, and ethylene dichloride and methanol.

### EXAMPLE 1

A therapeutic composition provided by the invention is prepared as follows: first, 25 mg of antidepressant amitriptyline hydrochloride granules are added to a spray coater. Then, an aqueous solution comprising 100 mg of pharmaceutically-acceptable coater polyoxyethylene of 100,000 molecular weight, and 5 mg of acacia binder is sprayed onto the drug to provide a wet mass of coated granules. Next, the coated granules are dried in a circulatory air current at 50°C to provide dry coated amitryptine. Then, the dry granules are added to a powder mixer and 0.1 mg of bulytparaben and 3 mg of magnesium stearate are added to the mixer and mixing continued to provide a homogenous, dry composition comprising coated amitryptline. The dry blend composition is compressed under a pressure head of two tons into a 9/32 inch (7.14 mm) diameter standard round tablet in a single-layered tablet press to provide a dosage form tablet comprising coated amitryptline. The dosage form tablet can be administered to a patient to provide a dose of amitryptyline.

### EXAMPLE 2

A dosage form adapted and shaped as a delivery tablet is manufactured as follows: first, 125 mg of polymer coated amitryptline prepared in Example 1 is added to a mixer. Then, 15 mg of osmagent sodium chloride is added to the mixer and the ingredients mixed to provide a homogenous composition. Next, 0.1 mg of bulytparaben, an antimicrobal preservative, and 3 mg of lubricant magnesium stearate are added to the mixer and mixing continued to provide a homogenous composition. The composition then is compressed under two tons of pressure into a round tablet to provide a compressed tablet core.

Next, the tablet core is coated with a wall comprising a semipermeable polymer composition. The wall-forming composition comprises 99% cellulose acetate having a 32.0% acetyl content, and 1% polyethylene glycol of 3,350 molecular weight. The wall forming composition is dissolved in an acetone: water (88:12 wt:wt) cosolvent to make a 5% solids solution. The wall-forming composition is sprayed onto the therapeutic tablet core in a coater. Then, one 25 mil (0.635 mm) exit passageway is drilled through the semipermeable wall to connect the drug with the exterior of the dosage form. The dosage form administered to a patient provides a sustained release of antidepressant therapy for 30 minutes up to 24 hours.

### EXAMPLE 3

A dosage form adapted, designed and shaped as an osmotic antidepressant delivery device is manufactured as follows: first, 670 g of the antidepressant amitriptyline hydrochloride and 303.8 g of poly(vinylpyrrolidone) were added to the bowl of a Glatt® Fluids Bed Granulator. The granulation process was then initiated by first fluidizing the dry antidepressant drug and the polymer and then spraying the blended granules with a binder solution. The binder solution was prepared by dissolving 6.2 g of poly(vinylpyrrolidone) in 148 g of water. The operating conditions during the aqueous poly(vinylpyrrolidone) spraying were as follows: solution spray rate of 16 g/min; inlet temperature 45°C; and outlet temperature of 22°C. During the granulating process, the filter bag was frequently shaken to remove any ungranulated materials. At the end of the solution spraying, the granulation was dried to reach a moisture content of less than 2% and removed from the fluid bed granulator The dried granulation was then screened and blended with 20 g of magnesium stearate in a Hobart® bowl mixer for 2 minutes.

Next, a push composition was prepared as follows: first, 415.5 g of pharmaceutically acceptable poly(ethylene oxide) comprising a 7,500,000 molecular weight, 150 g of sodium chloride, and 6 g of ferric oxide separately are screened through a 40 mesh screen. Then, all the screened ingredients are mixed with 30 g of hydroxypropylmethylcellulose comprising a 11,200 molecular weight to produce a homogenous blend. Next, 300 g of denatured anhydrous alcohol is added slowly to the blend with continuous mixing for 5 minutes. Then, the freshly prepared wet granulation is passed through a 20 mesh screen, allowed to dry at room temperature for 16 hours, and again passed through a 20 mesh screen. The screened granulation is mixed with 1.5 g of magnesium stearate in a rollermill for 5 minutes.

Next, the amitriptyline hydrochloride drug composition and the push composition are compressed using Manesty® BB₄ tablet press into bilayered cores of tablet shape, as follows: first, 123 mg of drug layer granulation is added to a punch and tamped, then, 90 mg of the push composition is added and the layers are pressed under a pressure head of 1,400 Ibs into a 5/16" diameter (7.97 mm) contacting layered arrangement.

The bilayered arrangements next are coated with a semipermeable wall. The wall-forming composition comprises 98% cellulose acetate having a 39.8% acetyl content, and 2% polyethylene glycol having a molecular weight of 3,350. The wall-forming composition is dissolved in acetone: water (95:5 wt:wt) cosolvent to make a 4% solid solution. The wall-forming composition is sprayed onto and around the bilayer cores in a 24" Vector Hi-Coater®.

Next, one 10 mil (0.254 mm) exit passageway is mechanically drilled through the semipermeable wall to connect the drug layer with the exterior of the dosage system. The residual solvent is removed by drying for 150 hours at 50°C and 50% humidity. Next, the osmotic systems are dried for 3 hours at 50°C to remove excess moisture. The dosage form produced by this manufacture provides 67 wt% amitriptyline, 31 wt% poly(vinylpyrrolidone) possessing 10,750 molecular weight, and 2 wt% magnesium stearate in drug composition. The push composition comprises 68.8% poly(ethylene oxide) comprising a 7,500,000 molecular weight, 25 wt% sodium chloride, 5 wt% hydroxypropylmethylcellulose possessing a 11,200 molecular weight, 1% ferric oxide and 0.25% magnesium stearate, 98 wt% cellulose acetate comprising a 39.8% acetyl content, and 2.0 wt% polyethylene glycol comprising a 3,350 molecular weight. The dosage form comprises one passageway, 10 mils (0.254 mm), and it has an amitriptyline release rate of 4.25 mg/h. The cumulative amount of amitriptyline release on 24 hours is depicted in drawing Figure 9. The dosage form delivered the drug free of blocking this passageway, thereby delivering the needed dose of drug to the patient.

### EXAMPLE 4

A dosage form adapted, designed and shaped as an osmotic antidepressant delivery device is manufactured as follows: first, 982.5 g of the antidepressant amitriptyline hydrochloride and 5.0 g of silicon dioxide was added to the bowl of a Glatt® Fluids Bed Granulator. The granulation process was then initiated by first fluidizing the dry antidepressant drug and the silicon dioxide and then spraying the blended granules with a binder solution. The binder solution was prepared by dissolving 2.5 g of poly(vinylpyrrolidone) in 150 g of water. The operating conditions during the aqueous poly(vinylpyrrolidone) spraying were as follows: solution spray rate of 15 g/min; inlet temperature 45°C; and outlet temperature of 23°C. During the granulating process, the filter bag was frequently shaken to remove any ungranulated materials. At the end of the solution spraying, the granulation was dried to reach a moisture content of less than 0.5% and removed from the fluid bed granulator. The dried granulation was then screened and blended with 10 g of magnesium stearate in a Hobart® bowl for 1 minute.

Next, a push composition was prepared as follows: first, 415.5 g of pharmaceutically acceptable poly(ethylene oxide) comprising a 7,500,000 molecular weight, 150 g of sodium chloride, and 6 g of ferric oxide separately are screened through a 40 mesh screen. Then, all the screened ingredients are mixed with 30 g of hydroxypropylmethylcellulose comprising a 11,200 molecular weight to produce a homogenous blend. Next, 300 g of denatured anhydrous alcohol is added slowly to the blend with continuous mixing for 5 minutes. Then, the freshly prepared wet granulation is passed through a 20 mesh screen, allowed to dry at room temperature for 16 hours, and again passed through a 20 mesh screen. The screened granulation is mixed with 1.5 g of magnesium stearate in a rollermill for 5 minutes.

Next, the amitriptyline hydrochloride drug composition and the push composition are compressed using Manesty® BB₄ tablet press into bilayered cores of tablet shape, as follows: first, 85 mg of the drug layer containing the amitriptyline is added to a punch and tamped, then, 60 mg of the push composition is added and the layers are pressed under a pressure head of 1,200 Ibs into a 9/32" diameter (0.7145 mm) contacting layered arrrangement.

The bilayered arrangements next are coated with a semipermeable wall. The wall-forming composition comprises 100% cellulose acetate having a 39.8% acetyl content. The wall-forming composition is dissolved in acetone: water (95:5 wt:wt) cosolvent to make a 4% solid solution. The wall-forming composition is sprayed onto and around the bilayer cores in a 24" Vector Hi-Coater®.

Next, the coated cores were divided into three parts and laser drilled to three different exit passageways. One set of cores comprised a 6 mil diameter (0.152 mm) passageway and the surface area of the passageway was 0.01824 mm². A second core set comprised a 10 mil (0.254 mm) diameter and the surface area of the passageway was 0.05067 mm². A third set of cores was laser drilled with an exit 15 mil in diameter (0.381 mm) and the surface area of the passageway was 0.1140 mm².

The residual solvent is removed from all the sets by drying for 200 hours at 50°C and 50% humidity. Next, the osmotic systems are dried an additional 4 hours at 50°C to remove excess moisture. The osmotic systems were placed into an artificial intestinal fluid, pH 7.5, at 37°C, and the release rate and the cumulative drug released were plotted as seen in Figures 6, 7, and 8. The osmotic systems produced by this manufacture provided 98.25 wt% amitriplytine hydrochloride, 0.25 wt% polyvinyl pyrrolidone possessing a 10,756 molecular weight, 0.50 wt% silicon dioxide and 1.0 at % magnesium stearate in the drug compartment. The push layer comprises the composition of Example 3.

### EXAMPLE 5

Following the procedure of Example 3, an osmotic dosage form comprising a dose of 50 mg of drug was prepared to provide the following dosage form: a drug layer comprising 67 wt% of amitriptyline, 5 wt% of poly(ethylene oxide) comprising a 100,000 molecular weight, 26.0 wt% of poly(vinyl pyrrolidone), and 2.0 wt% magnesium stearate. The push composition comprises 68.75 wt% poly(ethylene oxide), of 7,500,000 molecular weight, 25.0 wt% sodium chloride, 5.0 wt% hydroxypropylmethylcellulose, 1 wt% ferric oxide and 0.25 wt% magnesium stearate. The wall comprises 95 wt% cellulose acetate comprising 39.8% acetyl content and 5 wt% poly(ethylene glycol) of 3,350 molecular weight. The dosage form comprises one 10 mil orifice (0.254 mm) diameter, and the cumulative release rate is depicted in Figure 10. In this example, the drug layer weighed 82.1 mg consisting of 50 mg of amitriptyline and an amitriptyline overage of 5 mg. The dosage form has a 5.685 mg/h release rate and a nominal T-90 of 19.3 h.

### EXAMPLE 6

The procedure of Example 3 and Example 4 are followed in this example to provide a dosage form comprising a total drug content of 25 mg of amitriptyline-oxide, and a mean release rate of 2.85 mg/h.

### EXAMPLE 7

A dosage form adapted, designed and shaped as an osmotic drug delivery device is manufactured as follows: first, 12.5 kg of micronized imipramine, 11.25 kg of poly(ethylene oxide) possessing a 200,000 molecular weight are added to a Freund Flo-Coater's® bowl, a fluid bed granulator. The bowl was attached to the coater and granulation process was initiated for effecting granulation. Next, the dry powders were air suspended and mixed for 7 minutes. Then, a solution prepared by dissolving 1,000 g of poly(vinylpyrrolidone) identified as K29-32 having an average molecular weight of 40,000, in 15,667 g of water was sprayed from 3 nozzles onto the powder. The coating conditions were monitored during the process of aqueous poly(vinylpyrrolidone) as follows: solution spray rate of 125 g/min from each nozzle for a total spray rate of 375 g/min; inlet temperature 45°C; and process air flow of 1,000 cfm.

The coating process was computerized and automated in cycles. Each cycle contained 30 seconds of solution spraying followed by two seconds of drying and 10 seconds of filter bags shaking to unglue and possible powder deposits. At the end of the solution spraying, 16,667 g, the coated granulated particles were continued with the drying process for 25 minutes. The machine was turned off, and the coated granules were removed from the Flo-Coater. The coated granules were sized using a Fluid Air Mill. The granulation was transferred to a Rotocone®, mixed and lubricated with 237.5 g of magnesium stearate and 12.5 g of butyl hydroxytoluene.

Next, a push composition is prepared as follows: first, 4155.0 of pharmaceutically acceptable poly(ethylene oxide) comprising a 7,500,000 molecular weight, 1500 g of sodium chloride and 60 g of ferric oxide separately are screened through a 40 mesh screen. Then, all the screened ingredients are mixed with 300 g of hydroxypropylmethylcellulose comprising a 11,200 molecular weight to produce a homogenous blend. Next, 3000 g of denatured anhydrous alcohol is added slowly to the blend with continuous mixing for 5 minutes. The freshly prepared wet granulation is passed through a 20 mesh screen, allowed to dry at room temperature for 16 hours, and again passed through a 20 mesh screen. The screened granulation is mixed with 15 g of magnesium stearate in a rollermill for 5 minutes.

Next, the imipramine drug composition and the push composition are compressed into bilayered tablets. First, 352 mg of the imipramine composition is added to punch and tamped, then, 175 mg of the push composition is added and the layers are pressed under a pressure head of two tons into a 7/16" (1.11 cm) diameter contacting bilayered arrangements. The bilayered arrangements are coated with a semipermeable wall. The wall-forming composition comprises 95% cellulose acetate having a 39.8% acetyl content, and 5% polyethylene glycol having a molecular weight of 3,350. The wall-forming composition is dissolved in an acetone:water (90:10 wt:wt) cosolvent to make a 4% solids solution. The wall-forming composition is sprayed onto and around the bilayers in a 24" Vector Hi-Coater®.

Next, two 15 mil (0.381 mm) exit passageways are mechanically drilled through the semipermeable wall to connect the imipramine layer with the exterior of the dosage system. The residual solvent is removed by drying for 48 hours at 50°C and 50% humidity. Next, the osmotic systems are dried for 2 hours at 50°C to remove excess moisture. The dosage form produced by this manufacture provides 50 wt% imipramine, 45 wt% poly(ethylene oxide) possessing a 200,000 molecular weight, 4 wt% poly(vinyl pyrrolidone) possessing a 40,000 molecular weight, 0.95 wt% of magnesium stearate, and 0.5 wt% butyl hydroxytoluene. The push composition comprises 68.8 wt% poly(ethylene oxide) comprising a 7,500,000 molecular weight, 25 wt% sodium chloride, 5 wt% hydroxypropylmethylcellulose possessing 11,200 molecular weight, 1.0 wt% ferric oxide and 0.2 wt% magnesium stearate. The semipermeable wall comprises 95 wt% cellulose acetate comprising a 39.8% acetyl content, and 5.0 wt% polyethylene glycol comprising a 3,350 molecular weight.

### EXAMPLE 8

Following the procedure of Example 3, an osmotic dosage form comprising a drug dose of 80 mg of drug was prepared to provide the following dosage form: a drug layer comprising 50 wt% of desipramine, 25.05 wt% of poly(ethylene oxide) comprising a 200,000 molecular weight, 19.95 wt% of poly(ethylene oxide) comprising a 300,000 molecular weight, and 4.0 wt% of poly(vinyl pyrrolidone), and 1.0 wt% of magnesium stearate. The push composition comprises 68.75 wt% poly(ethylene oxide) of 7,500,000 molecular weight, 25.0 wt% sodium chloride, 5.0 wt% hydroxypropylmethylcellulose, 1 wt% ferric oxide and 0.25 wt% magnesium stearate. The wall comprises 95 wt% cellulose acetate comprising 39.8% acetyl content and 5 wt% poly(ethylene glycol) o 3,350 molecular weight. The dosage form comprises one 15 mils orifice, 0.114 m, and the drug layer consists of a dose of 80 mg of desipramine and releases desipramine average of 8 mg/hr.

### EXAMPLE 9

Scientific studies presented in the accompanying drawing figures evidence the unexpected, and improved results provided by the invention. In drawing Figure 11, the figure depicts the release rate in mg measured up to 24 hours from a dosage form comprising a 10 mil exit, (0.254 mm) with the dose released measured at pH 7.0 as indicated by a line with dark boxes, at pH 7.5 as indicated by a line with plus signs, at pH 8.0 as indicated by a line with stars, and at pH 8.5 as indicated by lines with clear boxes, which study evidence the sustained release of drug up to 24 hours in neutral to an increasing alkaline environment, such as the intestine and colon of the gastrointestinal tract. Drawing Figure 12 depicts the release rate in mg/hr over 24 hours as measured by two different procedures, wherein the line with dark boxes indicate the release rate measured by ultra violet detection and the line with crosses indicate the release rate measured by high pressure liquid chromatography detection, which results indicated the unexpected reproducibility of the invention.

### EXAMPLE 10

The procedures of the above examples are followed in this example, with the addition that the tricyclic antidepressant is a member selected from the groups consisting protriptyline, trimipramine, maprotiline, nortriptyline, imipramine, and the pharmaceutically acceptable salts.

### EXAMPLE 11

The procedures in the above examples are followed to provide a sustained-released dosage form comprising a drug core weighing 85 mg comprising 98.25 wt% amitriptyline hydrochloride, 0.25 wt% poly(vinylpyrrolidone), 1 wt% magnesium stearate, and 0.5 wt% silicon dioxide; an expansion composition weighing 60 mg comprising 63.75 wt% polyethylene of 7,000,000 molecular weight, 30 wt% sodium chloride, 5 wt% hydroxypropylmethylcellulose, 0.25 wt% magnesium stearate, and 1 wt% ferric oxide; a wall that surrounds the drug and expansion composition that weights 41 mg and comprises 100 wt% cellulose acetate comprising an acetyl content of 39.8%; a 10 mil (0.254 mm) exit in the wall; and a mean release rate of 3.783 mg/hr over 24 hours. The sustained release dosage form delivered 0 mg to 27 mg in 0 hours to 8 hours, 0 mg to 56 mg in 0 to 6 hours, and 0 mg to 68 mg in 0 hours to 24 hours.

### EXAMPLE 12

The procedures in the previous examples are followed in the manufacture of a sustained-release dosage form comprising the following: a drug core weighing 123.1 mg comprising 67 wt% amitriptyline hydrochloride, 31 wt% poly(vinyl-pyrrolidone) and 2 wt% magnesium stearate; an expandable-push composition comprising 68.75 wt% polyethylene oxide of 7,000,000 molecular weight, 25 wt% sodium chloride, 5 wt% hydroxypropylmethylcellulose, 0.25 wt% magnesium stearate, and 1 wt% ferric oxide; a wall that surrounds the drug core and the expandable composition, which wall weighs 38.5 mg and comprises 98 wt% cellulose acetate comprising 39.8 wt acetyl content and 2 wt% polyethylene glycol; a 10 mil (0.254 mm) exit in the wall; and an amitriptyline hydrochloride mean release rate of 4.2 mg/hr. The sustained release dosage form delivered amitriptyline hydrochloride at a cumulative amount released in mg of 0 mg to 22 mg in 0 hours to 6 hours, 0 mg to 46 mg in 0 hours to 12 hours, 0 mg to 65 mg in 0 hours to 18 hours, and 0 mg to 75 mg in 0 hours to 24 hours.

### EXAMPLE 13

The manufacturing procedures present above are followed in this example to provide a dosage form comprising: a drug layer weighing 40 mg comprising 67 wt% amitriptyline hydrochloride, 31 wt% polyvinylpyrrolidone, and 2 wt% magnesium stearate; an expandable-push layer weighing 22.1 mg comprising 63.75 wt% polyethylene oxide of 7,000,000 molecular weight, 30 wt% osmagent sodium chloride, 5 wt% hydroxypropylmethylcellulose of 11,300 molecular weight, 1 wt% ferric oxide and 0.25 wt% magnesium stearate; a wall that defines the dosage form and envelopes the drug layer and the expandable layer, which wall weighs 7.5 mg and comprises 50 wt% cellulose acetate comprising a 39.8 wt acetyl content, 48 wt% ethylcellulose, and 2 wt% polyethylene glycol; a 10 mil (0.254 mm) exit in the wall; and a mean release rate of 1.496 mg/hr. The dosage form releases 0 mg to 9 mg in 0 hours to 6 hours, 9 mg to 17 mg in 7 hours to 12 hours, 17 mg to 21 mg in 8 to 18 hours, and 21 mg to 23 mg in 19 to 22 hours.

### DISCLOSURE OF USE OF THE DOSAGE FORM FOR PERFORMING A METHOD OF PRACTICING THE INVENTION

An embodiment of the invention pertains to the use of the dosage form provided by the invention in a method for delivering a tricyclic antidepressant drug at a controlled rate orally to a warm-blooded animal in need of an antidepressant drug therapy, wherein the use comprises the steps of: (A) admitting into the warm-blooded animal a dosage form comprising: (1) a wall surrounding a compartment, the wall comprising a semipermeable polymeric composition permeable to the passage of fluid and substantially impermeable to the passage of drug; (2) a push layer in the compartment comprising an osmotic formulation for imbibing and absorbing fluid for expanding in size for pushing an antidepressant drug form the dosage form; (3) at least one osmotic dimensioned passageway in the wall for releasing the drug; and wherein the dosage form is characterized by (4) a drug layer in the compartment comprising a tricyclic antidepressant selected from the group consisting of protriptyline, maprotiline, trimipramine, notriptyline, amitriptylinoxide, imipramine, amitriptyline, desipramine, and imipramine oxide, for performing an antidepressant therapeutic program; (B) imbibing fluid through the semipermeable wall at a fluid rate determined by the permeability of the semipermeable wall and the osmotic pressure across the semipermeable wall causing the push layer to expand; and (C) delivering the therapeutically active antidepressant drug from the dosage form through the exit passageway to the warm-blooded animal over a prolonged period of time up to 24 hours. The antidepressant is administered by the dosage form for treating endogenous depression, depression accompanied by anxiety, for the treatment of mental depression, endogenous depression such as manic depressive reactions and reactive depressions, as secondary sedatives, and for treating agoraphobia and panic attacks.

Dosage form 10 of this invention can be used for administering the tricyclic antidepressant drug essentially-free of crystalline precipitation in the dosage form and without diminishing the dimensions of the exit means, during tricyclic antidepressant drug delivery over 30 hours. Dosage form 10, can be used in a method for administering a drug by the oral route, and in another method, the dosage form can be sized and shaped for administering a drug by the sublingual and buccal routes. The sublingual and buccal routes can be used for quicker therapy and they can be used when a smaller dose of drug is needed for therapy. The latter routes can be used as a by-pass of the first pass of hepatic metabolism of the drug. The antidepressant drugs can be administered by the sublingual or buccal routes and can be used for administering the drug for the management of depressant patients.

The method of the invention provides for delivering the antidepressant tricyclic drugs (see in accompanying drawing Figures 13, 14 and 15), wherein drug "a" is amitriptylinoxide, "b" is amitriptyline, "c" is trimipramine, "d" is maprotiline, "e" is protriptyline, "f" is desipramine, "g" is imipramine, "h" is nortriptyline, and "I" imipramine oxide.

In summary, it will be appreciated that the present invention contributes to the art an unobvious dosage form that possesses practical utility, can administer a drug at a dose metered release rate per unit time.

## Claims

1. A sustained release dosage form comprising 1 mg to 750 mg of a pharmaceutically acceptable encapsulated antidepressant tricyclic drug and a pharmaceutically acceptable carrier formulated for once-a-day oral administration to a patient in need of antidepressant therapy.

2. A sustained release dosage form according to claim 1, wherein the antidepressant tricyclic drug is selected from amitriptylinoxide, amitriptyline, trimipramine, maprotiline, protriptyline, desipramine, notriptyline and imipramine.

3. A sustained release dosage form according to either claim 1 or 2, wherein the pharmaceutically acceptable carrier is a hydrogel.

4. A sustained release dosage form according to any preceding claim, wherein the pharmaceutically acceptable carrier is polyalkylene oxide or carboxyalkylcellulose.

5. A sustained release dosage form according to any preceding claim, wherein the sustained release dosage form is formulated to administer a dose of the antidepressant tricyclic drug for up to thirty hours.

6. A sustained release dosage form according to any preceding claim, wherein the sustained release dosage form further comprises a binder and/or a lubricant.

7. A sustained release dosage form according to any preceding claim, wherein the antidepressant tricyclic drug is encapsulated by a member selected from poly-N-vinylamide, poly-N-vinylmethylacetamide, poly-N-vinylethylacetamide, poly-N-vinyl-methylpropionamide, poly-N-vinylethylpropionamide, poly-N-vinylmethylisobutyramide, poly-N-vinyl-2-pyrrolidone, polyvinylpyrrolidone, poly-N-vinyl-3-methyl-2-pyrrolidone, hydroxyalkylcellulose and hydroxypropylalkylcellulose.

8. A sustained release dosage form according to any preceding claim, wherein a wall comprising an exit passage and permeable to aqueous and biological fluids surrounds the encapsulated, antidepressant drug.

9. Use of a pharmaceutically acceptable encapsulated antidepressant tricyclic drug in the manufacture of a sustained release dosage form according to any preceding claim, for the treatment of a depression selected from endogenous depression, depression accompanied by anxiety, mental depression, manic depression reactions, reactive depressions and depressions accompanied by agoraphobia and panic attacks.

## Patentansprüche

1. Dosierungsform mit verzögerter Freisetzung, umfassend 1 mg bis 750 mg eines pharmazeutisch geeigneten, eingekapselten, tricyclischen Antidepressionsmittels und einen pharmazeutisch geeigneten Träger, die für eine orale Verabreichung einmal täglich an einen Patienten, der eine Therapie gegen Depressionen benötigt, formuliert ist.

2. Dosierungsform mit verzögerter Freisetzung nach Anspruch 1, worin das tricyclische Antidepressionsmittel ausgewählt ist aus Amitriptylinoxid, Amitriptylin, Trimipramin, Maprotilin, Protriptylin, Desipramin, Notriptylin und Imipramin.

3. Dosierungsform mit verzögerter Freisetzung nach einem der Ansprüche 1 oder 2, worin der pharmazeutisch geeignete Träger ein Hydrogel ist.

4. Dosierungsform mit verzögerter Freisetzung nach einem der vorhergehenden Ansprüche, worin der pharmazeutisch geeignete Träger ein Polyalkylenoxid oder Carboxyalkylcellulose ist.

5. Dosierungsform mit verzögerter Freisetzung nach einem der vorhergehenden Ansprüche, worin die Dosierungsform mit verzögerter Freisetzung so formuliert ist, daß sie eine Dosis des tricyclischen Antidepressionsmittels bis zu dreißig Stunden lang verabreichen kann.

6. Dosierungsform mit verzögerter Freisetzung nach einem der vorhergehenden Ansprüche, worin die Dosierungsform mit verzögerter Freisetzung weiterhin ein Bindemittel und/oder ein Gleitmittel umfaßt.

7. Dosierungsform mit verzögerter Freisetzung nach einem der vorhergehenden Ansprüche, worin das tricyclische Antidepressionsmittel eingekapselt ist von einem Mitglied, das ausgewählt ist aus Poly-N-vinylamid, Poly-N-vinylmethylacetamid, Poly-N-vinylethylacetamid, Poly-N-vinyl-methylpropionamid, Poly-N-vinylethylpropionamid, Poly-N-vinylmethylisobutyramid, Poly-N-vinyl-2-pyrrolidon, Polyvinylpyrrolidon, Poly-N-vinyl-3-methyl-2-pyrrolidon, Hydroxyalkylcellulose und Hydroxypropylalkylcellulose.

8. Dosierungsform mit verzögerter Freisetzung nach einem der vorhergehenden Ansprüche, worin eine Wand, die einen Ausgang umfaßt und durchlässig gegenüber wäßrigen und biologischen Flüssigkeiten ist, das eingekapselte Antidepressionsmittel umgibt.

9. Verwendung eines pharmazeutisch geeigneten, eingekapselten tricyclischen Antidepressionsmittels bei der Herstellung einer Dosierungsform mit verzögerter Freisetzung nach einem der vorhergehenden Ansprüche, zur Behandlung einer Depression, die ausgewählt ist aus endogener Depression, Depression in Begleitung von Angstzuständen, mentaler Depression, manischen Depressionsreaktionen, reaktiven Depressionen und Depressionen in Begleitung von Platzangst und Panikattacken.

## Revendications

1. Forme posologique à libération prolongée comprenant 1 mg à 750 mg d'un médicament antidépresseur tricyclique encapsulé, pharmaceutiquement acceptable, et un support pharmaceutiquement acceptable, formulée pour une administration orale une fois par jour à un patient nécessitant une thérapie par antidépresseur.

2. Forme posologique à libération prolongée selon la revendication 1, dans laquelle le médicament antidépresseur tricyclique est choisi parmi l'amitriptylinoxide, l'amitriptyline, la trimipramine, la maprotiline, la protriptyline, la désipramine, la notriptyline et l'imipramine.

3. Forme posologique à libération prolongée selon l'une des revendications 1 ou 2, dans laquelle le support pharmaceutiquement acceptable est un hydrogel.

4. Forme posologique à libération prolongée selon l'une quelconque des revendications précédentes, dans laquelle le support pharmaceutiquement acceptable est le poly(oxyde d'alkylène) ou la carboxyalkylcellulose.

5. Forme posologique à libération prolongée selon l'une quelconque des revendications précédentes, dans laquelle la forme posologique à libération prolongée est formulée pour administrer une dose du médicament antidépresseur tricyclique pendant une durée allant jusqu'à trente heures.

6. Forme posologique à libération prolongée selon l'une quelconque des revendications précédentes, dans laquelle la forme posologique à libération prolongée comprend en outre un liant et/ou un lubrifiant.

7. Forme posologique à libération prolongée selon l'une quelconque des revendications précédentes, dans laquelle le médicament antidépresseur tricyclique est encapsulé par un élément choisi parmi le poly-N-vinylamide, le poly-N-vinylméthylacétamide, le poly-N-vinyléthylacétamide, le poly-N-vinylméthylpropionamide, le poly-N-vinyléthylpropionamide, le poly-N-vinylméthylisobutyramide, la poly-N-vinyl-2-pyrrolidone, la polyvinylpyrrolidone, la poly-N-vinyl-3-méthyl-2-pyrrolidone, l'hydroxyalkylcellulose et l'hydroxypropylalkylcellulose.

8. Forme posologique à libération- prolongée selon l'une quelconque des revendications précédentes, dans laquelle une paroi comprenant un passage de sortie et perméable aux fluides aqueux et biologiques entoure le médicament antidépresseur encapsulé.

9. Utilisation d'un médicament antidépresseur tricyclique encapsulé, pharmaceutiquement acceptable, dans la fabrication d'une forme posologique à libération prolongée, telle que définie à l'une quelconque des revendications précédentes, pour le traitement d'une dépression choisie parmi une dépression endogène, une dépression accompagnée par une angoisse, une dépression mentale, des réactions maniaco-dépressives, des dépressions réactives et des dépressions accompagnées par une agoraphobie et des attaques de panique.
